# FASCICULE DE BREVET EUROPEEN

(11) **EP 1 892 229 B1**
(45) Date de publication et mention de la délivrance du brevet: **11.07.2012**
(21) Numéro de dépôt: 07290910.4
(22) Date de dépôt: 18.07.2007
(51) Int. Cl.: B01J 29/40, B01J 35/08, B01J 37/00, B01J 35/10

(54) **Procédé de production de propylène en presence d'un catalyseur macroporeux se presentant sous forme de billes spheriques**
Verfahren zur Herstellung von Propylen in Gegenwart eines makroporösen Katalysators in Form von sphärischen Kügelchen
Method of producing propylene in the presence of a macroporous catalyst present in the form of spherical balls

(30) Priorité: 24.08.2006 FR 0607494
(43) Date de publication de la demande: 27.02.2008
(73) Titulaire: IFP Energies nouvelles, 92852 Rueil-Malmaison Cedex (FR)
(72) Inventeur: Pigeat, Brigitte, 69005 Lyon (FR); Coupard, Vincent, 69120 Vaulx en Velin (FR); Maury, Sylvie, 69390 Charly (FR); Drouet, Serge, 69250 Montanay (FR)

(56) Documents cités:
- EP-A- 1 637 575
- EP-A- 1 777 284
- WO-A-01/04237
- WO-A-03/078364
- FR-A1- 2 879 620

## Description

### Domaine de l'invention

La présente invention se rapporte au domaine de la production de propylène à partir d'une charge hydrocarbonée comprenant au moins des oléfines ayant 4 atomes de carbone et au moins des oléfines ayant 5 atomes de carbone. Ladite charge hydrocarbonée provient avantageusement soit de la coupe oléfinique C4/C5 issue d'une unité de vapocraquage soit des coupes oléfiniques C4 et essence issues d'une unité de craquage catalytique fluide (FCC) soit encore d'un mélange desdites coupes issues du vapocraquage et du craquage catalytique fluide. Le procédé de production de propylène selon l'invention met en oeuvre au moins un catalyseur sous forme de billes sphériques préparé en présence d'un porogène de manière à créer des domaines macroporeux au sein de la porosité de chacune desdites billes.

### Etat de la technique antérieure

De nombreux brevets et publications se sont déjà intéressés à la production de propylène.
En particulier, le procédé divulgué dans la demande internationale WO 01/04237 ou EP-A-1 637 575 est un procédé de production de propylène, en une étape, réalisé à partir d'oléfines légères et utilisant un catalyseur comprenant une zéolithe ZSM-5. Ce procédé utilise une technologie en lit fluidisé laquelle est onéreuse du point de vue des investissements, et nécessite une conduite du procédé relativement délicate. Elle conduit également à des pertes notables de catalyseur par attrition. Les zéolithes de type structural MFI, en particulier la zéolithe ZSM-5, ont été fréquemment utilisées dans des catalyseurs pour la mise en oeuvre de procédé de production de propylène (WO 99/29805, EP-A-0.921.181, EP-A-0.921.179, EP-A-1.195.424). Elles présentent généralement un rapport Si/Al élevé (de 180 à 1000) pour limiter les réactions de transfert d'hydrogène responsables de la production de diènes et d'aromatiques.
De manière générale, même si l'ensemble de ces procédés de production de propylène décrits antérieurement conduit à des rendements en propylène satisfaisants, la production de sous-produits, en particulier l'essence et les composés en C4 riches en oléfines dont l'isobutène, n'est généralement pas négligeable au détriment de la sélectivité envers le produit désiré, à savoir le propylène.
La présente invention se propose de mettre en oeuvre un procédé de production de propylène limitant la production desdits sous-produits indésirables pour favoriser la sélectivité de la réaction envers le propylène et augmenter ainsi le rapport propylène/isobutène afin de satisfaire les tendances du marché. Le procédé de production de propylène selon l'invention présente également l'avantage d'être moins coûteux en terme de consommation énergétique par rapport aux procédés antérieurs : il a en effet été découvert, de manière surprenante, que pour parvenir à une production identique de propylène (rendement identique en propylène), le procédé selon l'invention nécessite, lorsque l'unité réactionnelle fonctionne en lit mobile, une quantité d'oléfines en C4/C5 recyclée bien moins importante que celle nécessitée par les procédés antérieurs.

### Description de l'invention

La présente invention a pour objet un procédé de conversion directe d'une charge hydrocarbonée comprenant au moins des oléfines ayant 4 atomes de carbone et au moins des oléfines ayant 5 atomes de carbone pour la production de propylène, ledit procédé comprenant le passage de ladite charge dans au moins une unité réactionnelle pourvue d'au moins un catalyseur se présentant sous forme de billes sphériques de diamètre compris entre 1 et 3 mm, chacune desdites billes sphériques comprend au moins une zéolithe et au moins un support à base d'alumine et présente une distribution poreuse telle que le volume macroporeux mesuré par porosimétrie au mercure est compris entre 0,10 et 0,20 ml/g et le volume mésoporeux mesuré par porosimétrie au mercure est compris entre 0,25 et 0,35 ml/g.

Le procédé selon l'invention vise la production de propylène à partir de la conversion d'une charge hydrocarbonée comprenant au moins des oléfines ayant 4 atomes de carbone par molécule et au moins des oléfines ayant 5 atomes de carbone par molécule. Le procédé selon l'invention est dit procédé de conversion directe de ladite charge car la transformation de ladite charge pour la production de propylène est réalisée en une seule étape au sein d'au moins une unité réactionnelle laquelle peut contenir plusieurs réacteurs de manière à maintenir la température de réaction constante ou à veiller à ce que la température ne dévie pas davantage de ± 20 °C par rapport à ladite température de réaction voulue.

Selon l'invention, ladite charge traitée dans l'unité réactionnelle pour la production de propylène provient avantageusement soit de la coupe oléfinique C4/C5 issue d'une unité de vapocraquage soit des coupes oléfiniques C4 et essence issues d'une unité de craquage catalytique fluide (FCC) soit encore d'un mélange desdites coupes issues du vapocraquage et du craquage catalytique fluide. Le vapocraquage correspond au craquage à la vapeur d'eau de coupes hydrocarbonées diverses, le plus souvent d'une coupe dite naphta de point d'ébullition compris entre 100°C et 350°C, et produit majoritairement des oléfines, essentiellement de l'éthylène et du propylène, mais aussi des oléfines à nombre d'atome de carbone plus élevé. La coupe oléfinique C4/C5 issue d'une unité de vapocraquage est généralement produite avec un rendement pouvant aller, selon les charges et les conditions opératoires, jusqu'à 10% poids, et n'est pas immédiatement valorisable. Elle constitue une charge de choix pour être convertie en propylène dans le procédé selon l'invention.

S'agissant de la coupe oléfinique C4 issue d'une unité de vapocraquage et utilisée avantageusement dans le procédé de l'invention, ladite coupe peut avantageusement être une coupe C4 brute issue de l'unité de vapocraquage et/ou une coupe oléfinique C4 obtenue après traitement d'une coupe C4 brute issue de l'unité de vapocraquage, ledit traitement consistant à extraire des composés dioléfiniques, en particulier du butadiène, présents dans ladite coupe C4 brute, par absorption dans un solvant. Ce type de procédé d'extraction de composés dioléfiniques, en particulier du butadiène, est décrit, par exemple dans l'ouvrage intitulé Procédés de pétrochimie, tome 1, chapitre III, page 224, 1985, édition Technip (A. Chauvel - G Lefebvre - L.Castex). Avant d'être introduite dans au moins ladite unité réactionnelle pourvue d'au moins dudit catalyseur et réalisant la transformation en propylène, ladite coupe oléfinique C4 issue d'une unité de vapocraquage est avantageusement introduite dans au moins une unité d'hydrogénation sélective des composés poly-insaturés de type diènes et acétyléniques résiduels. Le passage de ladite coupe oléfinique C4 issue d'une unité de vapocraquage dans ladite unité d'hydrogénation sélective permet non seulement la conversion des dioléfines en mono-oléfines et également l'élimination des composés acétyléniques lesquels sont convertis en mono-oléfines. Ladite étape d'hydrogénation sélective est facultative lorsque la coupe oléfinique C4 issue de l'unité de vapocraquage a préalablement été soumise à un traitement consistant à extraire des composés dioléfiniques, en particulier du butadiène, présents dans ladite coupe C4 brute, par absorption dans un solvant comme indiqué ci-dessus, et que l'effluent issu de ladite unité d'extraction présente une teneur en composés dioléfiniques inférieure à 10000 ppm. Lorsque la teneur en composés dioléfiniques dans la coupe oléfinique C4 issue de l'unité de vapocraquage à traiter est importante, c'est-à-dire généralement supérieure à 1,5 % massique, on effectue ladite étape d'hydrogénation sélective en utilisant plusieurs réacteurs en série, par exemple deux, l'effluent comportant les dioléfines non converties étant avantageusement, au moins en partie, recyclé à l'entrée de l'unité d'hydrogénation sélective afin de permettre également de contrôler l'échauffement global de la réaction.
L'unité d'hydrogénation sélective est opérée en présence d'hydrogène qui est introduit dans ladite unité selon une quantité variant de 5 % à 30 % molaire au dessus de la quantité stoechiométrique de la réaction, préférentiellement de 10 % à 20 % molaire au dessus de la quantité stoechiométrique, le ratio molaire H₂/(dioléines + acétyléniques) étant ainsi compris entre 1,05 et 1,30, préférentiellement entre 1,1 et 1,2.
Le(s) catalyseur(s) utilisé(s) pour la mise en oeuvre de l'étape d'hydrogénation sélective est(sont) généralement formé(s) d'au moins un métal du groupe VIII, préférentiellement le nickel ou le palladium, déposé sur au moins un support à base d'oxyde réfractaire tel que l'alumine. Lorsque ledit métal du groupe VIII est le palladium, la teneur en ce métal est avantageusement comprise entre 0,1% et 5 % poids, préférentiellement entre 0,2% et 0,6 % poids, du catalyseur. Lorsque ledit métal du groupe VIII est le nickel, la teneur en ce métal est avantageusement comprise entre 5% et 25 % poids, préférentiellement entre 7% et 20 % poids, du catalyseur. Ledit catalyseur présente une surface spécifique S_{BET} qui permet de limiter les réactions de polymérisation à la surface du catalyseur d'hydrogénation sélective, ladite surface étant comprise entre 5 et 140 m²/g.
La réaction d'hydrogénation sélective est préférentiellement conduite dans un ou plusieurs réacteur(s) à lit fixe, généralement à écoulement descendant pour le premier réacteur réalisant la réaction principale, c'est-à-dire réalisant au moins 60 % de la conversion totale, (c'est le cas lorsque l'effluent à convertir contient plus de 1,5 % poids de dioléfines), et généralement à co-courant ascendant pour les réacteurs secondaires réalisant la phase de finition de la réaction. Les conditions opératoires au sein de l'unité d'hydrogénation sélective sont choisies pour que l'effluent issu de ladite unité reste à l'état liquide : la température au sein de ladite unité est comprise entre 20°C et 150 °C, la pression totale est comprise entre 0,5 bars et 4 MPa. La vvh (rapport du débit volumique horaire à 15°C de charge fraîche liquide sur le volume de catalyseur chargé) est comprise entre 4 et 10 h⁻¹. La teneur en composés poly-insaturés (dioléfines et/ou acétyléniques) de l'effluent sortant de l'étape d'hydrogénation sélective est comprise entre 10 ppm et 4000 ppm et préférentiellement entre 50 ppm et 1000 ppm.

La coupe essence oléfinique issue d'une unité de vapocraquage et utilisée avantageusement dans le procédé de l'invention comprend majoritairement des composés aromatiques (benzène, toluène, xylènes, ethylbenzène), des cyclodioléfines et des dioléfines (principalement l'isoprène), des alkénylaromatiques (composés aromatiques présentant un groupement alkyl à insaturation tel que le styrène), des paraffines et des oléfines, les composés aromatiques représentant de 40 à 60 % poids de ladite coupe, les cyclodioléfines et les dioléfines (principalement l'isoprène) représentant de 20 à 30 % poids de ladite coupe. Les composés hydrocarbonés en C5 représentent de 10 à 40% poids de ladite coupe essence, de préférence de 20 à 25 % poids de ladite coupe essence. Les composés poly-insaturés (composés diéniques et acétyléniques), présents dans ladite coupe essence oléfinique initiale, sont préférentiellement éliminés, en partie au moins, par passage de ladite coupe dans au moins une unité d'hydrogénation sélective, laquelle est pourvue d'au moins un catalyseur généralement formé d'au moins un métal du groupe VIII, préférentiellement le nickel ou le palladium, déposé sur au moins un support à base d'oxyde réfractaire tel que l'alumine. Lorsque ledit métal du groupe VIII est le palladium, la teneur en ce métal est avantageusement comprise entre 0,1% et 5 % poids, préférentiellement entre 0,2% et 0,6 % poids, du catalyseur. Lorsque ledit métal du groupe VIII est le nickel, la teneur en ce métal est avantageusement comprise entre 5% et 25 % poids, préférentiellement entre 7% et 20 % poids. Ledit catalyseur présente une surface spécifique S_{BET} qui permet de limiter les réactions de polymérisation à la surface du catalyseur d'hydrogénation sélective, ladite surface étant comprise entre 5 et 140 m²/g.
L'unité d'hydrogénation sélective est opérée en présence d'hydrogène lequel est introduit dans ladite unité selon une quantité variant de 100 à 500 normaux m³ de gaz / m³ de charge liquide à 15°C. La quantité d'hydrogène utilisée est excédentaire afin de favoriser la conversion d'espèces peu réactives, par exemple les composés styréniques, présentes dans ladite coupe essence oléfinique issue de l'unité de vapocraquage.
La réaction d'hydrogénation sélective est préférentiellement conduite dans un ou plusieurs réacteur(s) à lit fixe, généralement à écoulement descendant pour le premier réacteur réalisant la réaction principale, c'est-à-dire réalisant au moins 60 % de la conversion totale, (c'est le cas lorsque l'effluent à convertir contient plus de 1,5 % poids de dioléfines). Les conditions opératoires au sein de l'unité d'hydrogénation sélective sont choisies pour que l'effluent issu de ladite unité reste à l'état liquide : la température au sein de ladite unité est comprise entre 20°C et 200 °C, la pression totale est comprise entre 0,5 et 4 MPa et la pression d'hydrogène est inférieure à 2 MPa. La wh (rapport du débit volumique horaire à 15°C de charge fraîche liquide sur le volume de catalyseur chargé) est comprise entre 0,3 et 6 h⁻¹. L'effluent sortant de ladite unité d'hydrogénation sélective est avantageusement introduit dans au moins une colonne de séparation permettant de récupérer une fraction légère formée essentiellement (entre 10 et 40% de la coupe essence initiale) de composés à 5 atomes de carbone dont au moins 60% poids de mono-oléfines en C5 (pentène et isopentène), ladite fraction légère pouvant également contenir des paraffines en C5 ainsi que des hydrocarbures en C6 et C4. Une fraction lourde formée majoritairement de composés aromatiques (benzène, toluène, xylène, ethylbenzène), de composés oléfiniques (oléfines en C6), et composés cyclo-oléfiniques (dihydro-dicyclopentadiene et dérivés alkylés) est extraite en queue de la colonne de séparation. Ladite fraction légère est celle qui est utilisée pour la mise en oeuvre du procédé selon l'invention.

Le FCC désigne le craquage catalytique en lit fluidisé de fractions pétrolières de point d'ébullition supérieur à environ 350°C, généralement d'un distillat sous vide, éventuellement de l'huile désasphaltée ou d'un résidu atmosphérique. L'essence issue d'un FCC correspond à une coupe de point d'ébullition généralement compris entre 20°C et 250°C. Cette essence est relativement riche en composés insaturés de type oléfinique, mono-oléfines et di-oléfines, (entre 20 et 50 % poids), et contient du soufre jusqu'à des valeurs pouvant atteindre quelques % poids. Ladite coupe essence issue d'un FCC, dont le tiers en masse est constitué d'une coupe bouillant entre 20 et 60°C, est traitée dans au moins une unité d'hydrogénation sélective, notée SHU. Une séparation des composés légers, ou étêtage, est réalisée à la suite de l'étape SHU afin d'extraire une coupe bouillant entre 20 et 60°C et comprenant majoritairement, c'est-à-dire au moins 15 % poids, de préférence au moins 30% poids, de composés hydrocarbonés à 5 atomes de carbone. Cette coupe légère est riche en mono-oléfines (pentène et isopentène) et constitue une charge de choix pour la mise en oeuvre du procédé selon l'invention. Le(s) catalyseur(s) utilisé(s) pour la mise en oeuvre de l'étape d'hydrogénation sélective (SHU) est(sont) généralement formé(s) d'au moins un métal du groupe VIII, préférentiellement le nickel ou le palladium, déposé sur au moins un support à base d'oxyde réfractaire tel que l'alumine. Lorsque ledit métal du groupe VIII est le palladium, la teneur en ce métal est avantageusement comprise entre 0,1% et 5 % poids, préférentiellement entre 0,2% et 0,6 % poids, du catalyseur. Lorsque ledit métal du groupe VIII est le nickel, la teneur en ce métal est avantageusement comprise entre 5% et 25 % poids, préférentiellement entre 7% et 20 % poids et le catalyseur est sulfuré afin de passiver les atomes de nickel en surface. Ledit catalyseur d'hydrogénation sélective présente une surface spécifique S_{BET} qui permet de limiter les réactions de polymérisation à la surface du catalyseur d'hydrogénation sélective, ladite surface étant comprise entre 5 et 140 m²/g. De l'hydrogène est introduit dans l'unité d'hydrogénation sélective selon une quantité variant de 5 % à 30 % molaire au dessus de la quantité stoechiométrique de la réaction, préférentiellement de 10 % à 20 % molaire au dessus de la quantité stoechiométrique. Les conditions opératoires au sein de l'unité d'hydrogénation sélective sont choisies pour que l'effluent issu de ladite unité reste à l'état liquide : la température au sein de ladite unité est comprise entre 120°C et 200 °C, la pression totale est comprise entre 0,5 et 4 MPa. La vvh (rapport du débit volumique horaire à 15°C de charge fraîche liquide sur le volume de catalyseur chargé) est comprise entre 4 et 10 h⁻¹.

La coupe C4 issue du FCC correspond à une fraction hydrocarbonée composée majoritairement, c'est-à-dire au moins 80 % poids, des molécules à 4 atomes de carbone produites par le FCC. Cette coupe représente généralement 4 à 10 % poids de la charge initiale traitée par le FCC. Elle comprend au moins 30% poids d'oléfines, de préférence au moins 60 % poids, la proportion de mono-oléfines étant majoritaire par rapport aux di-oléfines. Les mono-oléfines représentent généralement au moins 98% de la fraction oléfinique totale. Ladite coupe C4 issue du FCC contient également des hétéro-éléments, généralement du soufre présent en une quantité représentant de 5 à 50 ppm, en particulier sous forme de mercaptan, et/ou de l'azote sous forme ammoniaque et/ou acétonitrile, présent généralement en une quantité représentant de 5 à 30 ppm. Les composés soufrés, en particulier sous forme de mercaptans sont, en partie au moins, extraits de ladite coupe issue du FFC dans laquelle ils sont présents par traitement de ladite coupe dans au moins une unité de contactage consistant à mettre en contact de la soude avec ladite coupe contenant lesdits composés soufrés, au moins une unité de lavage à l'eau et au moins un coallesceur étant situés en aval de ladite unité de contactage. L'unité de contactage est pourvue par exemple de Merox^{™} extractif ou de sulfrex^{™} ; il s'agit de colonne d'adsorption contenant une solution aqueuse de soude ayant une concentration voisine de 10 % molaire. Cette étape est réalisée sous une pression suffisante pour que les réactifs restent liquides et à une température comprise entre 40 et 100 °C. L'effluent issu de l'étape de contactage ne contient pas, préférentiellement, davantage que 3 ppm de soufre. Il constitue avantageusement, en partie au moins, la charge introduite dans la zone réactionnelle réalisant la transformation en propylène. L'unité de lavage à l'eau, placée en aval de l'unité de contactage, réalise l'élimination d'au moins 80 % poids des espèces azotées présentes dans la coupe C4 issue du FCC. L'étape de contactage est particulièrement avantageuse lorsque la teneur en soufre de la coupe C4 issue du FCC est au moins égale à 5 ppm.
L'effluent appauvri en composés soufrés est ensuite avantageusement introduit au moins dans une unité d'hydrogénation sélective. De l'hydrogène est introduit dans l'unité d'hydrogénation sélective selon une quantité variant de 5 % à 30 % molaire au dessus de la quantité stoechiométrique de la réaction, préférentiellement de 10 % à 20 % molaire au dessus de la quantité stoechiométrique, le ratio molaire H₂/dioléfines étant ainsi compris entre 1,05 et 1,3 préférentiellement entre 1,1 et 1,2.
Le(s) catalyseur(s) utilisé(s) pour la mise en oeuvre de l'étape d'hydrogénation sélective est(sont) généralement formé(s) d'au moins un métal du groupe VIII, préférentiellement le nickel ou le palladium, déposé sur au moins un support à base d'oxyde réfractaire tel que l'alumine. Lorsque ledit métal du groupe VIII est le palladium, la teneur en ce métal est avantageusement comprise entre 0,1% et 5 % poids, préférentiellement entre 0,2% et 0,6 % poids, du catalyseur. Lorsque ledit métal du groupe VIII est le nickel, la teneur en ce métal est avantageusement comprise entre 5% et 25 % poids, préférentiellement entre 7% et 20 % poids. Ledit catalyseur présente une surface spécifique S_{BET} qui permet de limiter les réactions de polymérisation à la surface du catalyseur d'hydrogénation sélective, ladite surface étant comprise entre 5 et 140 m²/g.
La réaction d'hydrogénation sélective est préférentiellement conduite dans un ou plusieurs réacteur(s) à lit fixe, généralement à écoulement descendant pour le premier réacteur lorsque l'effluent à convertir, appauvri en composés soufrés, contient plus de 1,5 % poids de dioléfines, et généralement à co-courant ascendant pour les réacteurs secondaires lorsque ledit effluent à convertir, appauvri en composés soufrés, contient moins de 1,5 % poids de dioléfines. Les conditions opératoires au sein de l'unité d'hydrogénation sélective sont choisies pour que l'effluent issu de ladite unité reste à l'état liquide : la température au sein de ladite unité est comprise entre 20°C et 150 °C, la pression totale est comprise entre 0,5 à 4 MPa. La vvh (rapport du débit volumique horaire à 15°C de charge fraîche liquide sur le volume de catalyseur chargé) est comprise entre 4 et 10 h⁻¹.

La charge hydrocarbonée comprenant au moins des oléfines ayant 4 atomes de carbone et au moins des oléfines ayant 5 atomes de carbone et introduite dans ladite unité réactionnelle, mise en oeuvre pour la production de propylène, est composée majoritairement de composés mono-oléfiniques à 4 et 5 atomes de carbone, c'est-à-dire de 20 à 100% poids, de préférence de 25 à 60 % poids de composés mono-oléfiniques à 4 et 5 atomes de carbone. Ladite charge hydrocarbonée peut également contenir des composés dioléfiniques à 4 et/ou 5 atomes de carbone ne représentant préférentiellement pas plus de 1 % poids de ladite charge hydrocarbonée entrant dans ladite zone réactionnelle. Des composés oléfiniques ayant davantage que 5 atomes de carbone par molécule peuvent également être présents. Des composés ayant au moins un hétéro-élément, notamment des composés soufrés, peuvent être présents en faible quantité : ils ne représentent pas davantage que 100 ppm de ladite charge hydrocarbonée entrant dans ladite zone réactionnelle.

Une charge présentant une telle composition est avantageusement obtenue par mélange d'une coupe C4 et d'une coupe C5 issues d'une unité de vapocraquage, par mélange d'une coupe C4 et d'une coupe essence issues d'une unité de craquage catalytique fluide (FCC), par mélange d'une coupe C4 issue d'une unité de vapocraquage et d'une coupe essence issue d'une unité de craquage catalytique fluide (FCC), par mélange d'une coupe C4 issue d'une unité de craquage catalytique fluide (FCC) et d'une coupe C5 issue d'une unité de vapocraquage ou encore par mélange des coupes C4/C5 issues d'une unité de vapocraquage et des coupes C4 et essence issues d'une unité de craquage catalytique fluide (FCC), lesdites coupes issues d'une unité de vapocraquage et celles issues d'une unité de craquage catalytique présentent les caractéristiques des coupes décrites ci-dessus et préalablement traitées selon les procédés décrits ci-dessus (hydrogénation sélective, procédé de contactage).

L'unité réactionnelle réalisant la conversion de ladite charge hydrocarbonée pour la production de propylène et pourvue d'au moins dudit catalyseur est mise en oeuvre à une température comprise entre 450 et 580°C, à une pression opératoire comprise entre 0,01 et 0,5 MPa et une pph (débit massique horaire de charge / masse du catalyseur) comprise entre 1 et 20 h⁻¹. Ledit catalyseur est mis en oeuvre dans ladite unité réactionnelle fonctionnant soit en lit mobile soit en lit fixe, préférentiellement en lit mobile.

L'effluent issu de ladite unité réactionnelle est fractionné de manière à recueillir séparément au moins une première fraction contenant le propylène recherché, au moins une deuxième fraction renfermant de l'hydrogène, au moins une troisième fraction renfermant des composés hydrocarbonés non aromatiques à 4, 5 et/ou 6 atomes de carbone par molécule et au moins une quatrième fraction renfermant des composés aromatiques à au moins 6 atomes et/ou des composés hydrocarbonés lourds ayant au moins 7 atomes de carbone par molécule et généralement au moins 9 atomes de carbone par molécule.

Ladite première fraction contenant le propylène renferme également, généralement, de 5 à 7 % poids de propane. Une unité de séparation du propylène est avantageusement placée en aval de la zone de récupération de ladite première fraction de manière à obtenir une fraction hydrocarbonée dont la teneur en propylène est augmentée. Ladite deuxième fraction est constitué d'hydrogène, présent dans une proportion représentant au moins 7 % poids de ladite deuxième fraction, laquelle contient également avantageusement au moins 60 % poids d'éthylène qui peut être isolé de ladite deuxième fraction afin d'être valorisé comme intermédiaire pétrochimique. Ladite deuxième fraction est avantageusement recyclée en amont de ladite unité réactionnelle afin d'accroître la quantité de propylène formé. Ladite troisième fraction, renfermant des composés hydrocarbonés non aromatiques à 4, 5 et/ou 6 atomes de carbone par molécule, est avantageusement recyclée, en partie au moins, en amont de ladite unité réactionnelle lorsque celle-ci fonctionne en lit mobile. Elle contient les composés oléfiniques qui n'ont pas réagi. Elle contient aussi des paraffines inertes dont le recyclage à l'entrée de la zone réactionnelle permet d'accroître la sélectivité du catalyseur envers le propylène par baisse de pression partielle d'oléfine. De manière préférée, la proportion de ladite troisième fraction qui est recyclée est telle que le rapport {débit massique de la troisième fraction en entrée de ladite zone réactionnelle/débit massique de la charge hydrocarbonée fraîche entrant dans ladite unité réactionnelle avant mélange avec les fractions recyclées}, appelé taux de recyclage, est compris entre 0,5 et 2, de préférence entre 0,5 et 1,5. Le recyclage d'une partie au moins de ladite troisième fraction permet, en partie au moins, de préférence en totalité, la ré-introduction des oléfines non converties dans ladite unité réactionnelle ainsi que des produits secondaires non désirés formés au cours de la réaction de conversion et qu'il convient de convertir en propylène. Un taux de recyclage important entraîne une consommation énergétique élevée et est donc préjudiciable pour la viabilité économique du procédé d'autant plus qu'un taux de recyclage élevé, à savoir supérieur à 1,5 préférentiellement supérieur à 2 est la conséquence d'un catalyseur généralement peu actif.

Le catalyseur, employé dans chacun des réacteurs de l'unité réactionnelle du procédé de l'invention, se présente sous forme de billes sphériques ayant une double porosité mesurée par porosimétrie au mercure : une macroporosité caractérisée par un volume mercure macroporeux compris dans une gamme variant de 0,10 à 0,20 ml/g et de préférence dans une gamme variant de 0,12 à 0,18 ml/g et une mésoporosité caractérisée par un volume mercure mésoporeux compris dans une gamme variant de 0,25 à 0,35 ml/g de préférence dans une gamme variant de 0,28 à 0,35 ml/g. La macroporosité est également caractérisée par la présence de domaines macroporeux au delà de 50 nm, de préférence au delà de 100 nm et/ou résulte d'une macroporosité texturale intraparticulaire, la mésoporosité est aussi caractérisée par la présence de domaines mésoporeux compris dans une gamme variant de 7 à 50 nm et de préférence dans une gamme variant de 8 à 10 nm. La proportion du volume poreux desdites billes ayant une taille de pores inférieure à 20 nm est comprise entre 60 et 70 %.
L'analyse porosimétrie au mercure correspond à l'intrusion d'un volume de mercure caractéristique de l'existence de mésopores et de macropores dans ledit catalyseur selon la norme ASTM D4284-83 à une pression maximale de 4000 bars, utilisant une tension de surface de 484 dynes/cm et un angle de contact de 140° (valeur choisie suivant les recommandations de l'ouvrage "Technique de l'ingénieur, traité analyse et caractérisation", page 1050, écrit par J. Charpin et B. Rasneur) et les pores étant supposés de forme cylindrique. Cette technique permet d'accéder à la valeur du volume mercure mésoporeux défini comme étant le volume mercure adsorbé par l'ensemble des pores ayant un diamètre compris dans la gamme des mésopores à savoir compris entre 3,6 et 50 nm. De même, le volume mercure macroporeux est défini comme étant le volume mercure adsorbé par l'ensemble des pores ayant un diamètre supérieur à 50 nm.

Conformément à l'invention, la zéolithe présente dans chacune desdites billes sphériques formant le catalyseur est préférentiellement choisie parmi les zéolithes de type structural MEL, MFI, NES, EUO, FER, CHA, MFS, MWW et NES et très préférentiellement il s'agit d'une zéolithe de type structural MFI, en particulier la zéolithe ZSM-5. Ladite zéolithe peut également être avantageusement choisie parmi les zéolithes NU-85, NU-86 et IM-5. De manière avantageuse, ladite zéolithe présente dans chacune desdites billes sphériques formant le catalyseur a un rapport Si/Al compris entre 50 et 500 et très avantageusement compris entre 70 et 140. Ladite zéolithe est dispersée dans un support à base d'alumine au sein de chacune desdites billes sphériques formant le catalyseur. La proportion de zéolithe dans chacune desdites billes formant le catalyseur est comprise entre 15 et 90 % poids, de préférence entre 30 et 80 % poids et de manière très préférée entre 35 et 50% poids, le reste étant constitué du support à base d'alumine.

Conformément à l'invention, lesdites billes sphériques constituant ledit catalyseur employé dans le procédé de l'invention ont un diamètre compris entre 1 et 3 mm, de préférence compris entre 1,8 et 2,2 mm. La morphologie et la distribution en taille des billes sont établies par analyse de photos obtenues par microscopie électronique à balayage (MEB). Le catalyseur présente une surface spécifique S_{BET} comprise entre 290 et 350 m²/g. Il présente une résistance mécanique mesurée par l'écrasement grains à grains, selon la méthode ASTM D4179-88a, telle que EGG soit au moins égal à 10 N et de préférence au moins égal à 20 N.

Le catalyseur est préparé selon un procédé comprenant a) la préparation d'au moins une émulsion formée d'au moins un porogène, de l'eau et d'un tensioactif, b) la préparation d'une suspension formée d'eau, d'acide, d'une source d'alumine, d'au moins une zéolithe et de ladite émulsion préparée au cours de l'étape a), c) la mise en forme par coagulation en gouttes consistant i) à faire passer ladite suspension formée en b) dans un pot d'égouttage constitué de buses ayant chacune un orifice de taille calibrée de manière à former des gouttelettes, ii) à faire passer, selon un mouvement descendant, lesdites gouttelettes dans une colonne contenant une phase supérieure constituée par une phase organique et une phase inférieure constituée par une phase aqueuse basique, l'interface phase organique - phase aqueuse étant constitué d'un tensio-actif, de manière à récolter des billes sphériques, d) le séchage desdites billes et e) la calcination desdites billes.

Pour la préparation de l'émulsion selon l'étape a), le porogène, utilisé pour former des pores dans les billes du catalyseur final, est une coupe pétrolière, préférentiellement une coupe kérosène paraffinique ayant de 10 à 14 atomes de carbone, formée de normale et d'iso-paraffines, et présentant un point d'ébullition compris entre 220 et 350°C. On utilise avantageusement en tant que porogène un composé commercial, l'isane^{®}, dont la composition comporte plusieurs composants aromatiques. Le tensio-actif, utilisé pour la préparation de l'émulsion, est un agent émulsionnant non ionique. Il est choisi de façon à assurer la stabilité de l'émulsion. Il est essentiel qu'il puisse être éliminé par combustion et qu'il soit liquide à température ambiante. On choisit généralement comme tensio-actif un composé commercialisé, le Galoryl^{®}, vendu par le Comptoir Français des Produits Industriels. Le mélange de l'eau, du porogène et du tensio-actif est réalisé à température ambiante pendant une durée préférentiellement comprise entre 10 et 15 minutes.

La préparation de la suspension selon l'étape b) consiste, dans un premier temps, à mélanger l'eau, l'acide et la source d'alumine puis à introduire dans le mélange ainsi formé au moins une zéolithe et enfin à y introduire l'émulsion formée au cours de l'étape
a). Le mélange d'eau, d'acide et de la source d'alumine est réalisé à température ambiante. L'eau et l'acide sont mélangés simultanément puis la source d'alumine est introduite. L'acide employé pour la préparation de la suspension est avantageusement choisi parmi les acides forts, de préférence l'acide nitrique et l'acide sulfurique. On utilise très avantageusement l'acide nitrique et en particulier l'acide nitrique à 59,68% poids. De manière préférée, on utilise un mélange d'acide nitrique et d'acide phosphorique La source d'alumine, employée pour la préparation de la suspension, est de préférence choisie dans le groupe formé par l'hydrargillite, la bayerite, la pseudo-boehmite, les gels amorphes, les alumines dites de transition qui comportent au moins une phase prise dans le groupe comprenant les phases rhô, chi, eta, gamma, kappa, theta et alpha. De manière très préférée, ladite source d'alumine est une pseudo-boehmite, par exemple le PURAL SB3^{®} commercialisé par la société SASOL. La préparation de la suspension est poursuivie en introduisant au moins une zéolithe, sous forme de poudre, dans le mélange contenant l'eau, l'acide et la source d'aluminium, à température ambiante. La zéolithe employée pour la préparation de la suspension peut se trouver indifféremment sous sa forme brute de synthèse, sous forme échangée ou sous forme calcinée (forme hydrogène). La préparation de la suspension se termine par l'introduction de l'émulsion préparée au cours de l'étape a) dans le mélange {eau, acide, source d'alumine, zéotithe}. Ladite suspension est agitée vigoureusement jusqu'à ce que la viscosité de ladite suspension soit comprise entre 250 et 400 mPa.s.. L'agitation vigoureuse est réalisée préférentiellement entre 1100 et 1900 tours/min et très préférentiellement entre 1400 et 1700 tours/min pendant une dizaine de minutes, généralement entre 10 et 15 minutes puis la vitesse d'agitation est réduite pour être comprise préférentiellement entre 550 et 700 trs/min jusqu'à ce que la viscosité de ladite suspension soit comprise entre 250 et 400 mPa.s. Ainsi, la suspension présente les propriétés rhéologiques adaptées pour être écoulée au travers des buses du pot d'égouttage employé au cours de l'étape c) de mise en forme du catalyseur par coagulation en gouttes. La viscosité de ladite suspension est mesurée au moyen d'un rhéomètre plan-plan pour un gradient de vitesse de cisaillement de 100 s⁻¹. La viscosité mesurée est la viscosité relative.

Les vitesses d'agitation sont celles obtenues au moyen d'un agitateur ER550 de la société Euromélanges. Le moteur fonctionne à courant continu monophasé de 220 volts, la puissance est égale à 0,55 kW à 3000 trs/min.

Selon le procédé de préparation du catalyseur employé dans le procédé de conversion selon l'invention, les quantités des différents réactifs présents dans l'émulsion et dans la suspension sont telles que :
- le taux de porogène égal à la masse de porogène sur la masse de l'eau engagée dans l'émulsion et de l'eau engagée dans la suspension est compris entre 1,5 et 8 % massique, de préférence entre 2 et 7,5% massique. L'eau présente dans les composés engagés dans l'émulsion et la suspension, notamment la source d'alumine et la zéolithe, n'intervient pas pour le calcul du taux de porogène.
- la proportion de tensio-actif, présent dans l'émulsion, et calculée comme étant égale à la masse de tensio-actif sur la masse de porogène est comprise entre 1 et 10 % massique, de préférence entre 4 et 8 % massique et de manière très préférée entre 5 et 7 % massique, 7 étant exclu de la gamme 5-7.
- la proportion d'eau présente dans la suspension (après l'introdusction de l'émulsion dans la suspension) est telle que le rapport masse sèche (correspondant à la masse de poudre, à savoir la source d'alumine et la zéolithe, déshydratée) sur la masse d'eau totale est comprise entre 20 et 30 % massique, de préférence entre 24 et 28 % massique.
- la quantité d'eau, engagée dans l'émulsion, représente de 9 à 11 % poids de la quantité d'eau totale engagée dans la suspension.
- le taux d'acide, engagé dans la suspension, égal au produit de la concentration (% poids) dudit acide par la masse dudit acide rapporté à la masse sèche de la source d'alumine est compris entre 10 et 15 % massique.
- la proportion de zéolithe, présente dans la suspension, et calculée comme étant égale au rapport de la masse sèche de la zéolithe sur la masse sèche de la source d'alumine et de la zéolithe est comprise entre 10 et 55 % massique, de préférence entre 30 et 55 % massique et de manière très préférée entre 35 et 50 % massique.
- la proportion de l'acide phosphorique, avantageusement introduit avec l'acide nitrique, est tel que le rapport massique P₂O5 / source d'alumine séchée est compris entre 1 et 5% massique.

La masse sèche de la source d'alumine et celle de la zéolithe sont accessibles par mesure de la perte au feu (PAF) de chacune de ces poudres.

La mise en forme par coagulation en gouttes consiste au cours d'une première étape i) à faire passer ladite suspension préparée au cours de l'étape b) dans un pot d'égouttage constitué de buses, chacune desdites buses ayant un orifice de taille calibrée de manière à former des gouttelettes. Ledit pot d'égouttage est placé en tête d'une colonne contenant une phase supérieure constituée par une phase organique et une phase inférieure constituée par une phase aqueuse basique, l'interface phase organique - phase aqueuse étant constitué d'un tensio-actif. Lesdites buses présentent chacune un orifice de taille calibrée de manière à former des gouttelettes de diamètre compris entre environ 2 et 3 mm. La dimension des gouttelettes obtenues dépend non seulement du diamètre intérieur des buses (phénomène de mouillage), lequel est généralement voisin de 1 mm, mais également de la forme à section circulaire desdites buses à leurs extrémités. Les gouttelettes ainsi formées traversent, conformément à l'étape ii) de la mise en forme par coagulation en gouttes, selon un mouvement descendant, ladite colonne contenant une phase supérieure constituée par une phase organique et une phase inférieure constituée par une phase aqueuse, l'interface phase organique - phase aqueuse étant constitué d'un tensio-actif, de manière à récolter des billes sphériques ayant un diamètre compris entre environ 2 et 3 mm. Ladite phase organique est choisie d'une manière telle qu'elle présente une masse volumique légèrement inférieure à celle de l'eau. De manière préférée, la phase organique est choisie de manière à ce que la masse volumique soit comprise entre 0,7 et 0,9 kg.m⁻³ à 15 °C. Ladite phase organique est choisie d'une manière telle que la tension superficielle entre ladite phase organique et ladite phase aqueuse basique soit élevée, généralement comprise 60.10⁻³ et 80.10⁻³ N/m. Avantageusement une coupe pétrolière, de préférence une coupe kérosène paraffinique, en particulier l'isane®, est choisie comme phase organique. Le tensio-actif séparant les phases organique et aqueuse est de préférence un TA cationique. De préférence, on utilise l'ammonyl BR 1244^{™}, un bromure d'alkyle diméthyl benzylammonium en solution aqueuse, commercialisé par la société SEPIC SA. La phase aqueuse basique constituant la partie inférieure de la colonne est avantageusement une solution basique ayant une concentration d'ammoniaque comprise entre 25 et 33 g.l⁻¹, de préférence entre 27 et 29 g.l⁻¹. Ladite solution aqueuse basique présente un pH compris entre 8 et 10.

La colonne, mise en oeuvre pour la mise en forme par coagulation en gouttes, est préparée en y introduisant d'abord ladite phase organique, préférentiellement l'isane®, puis ladite solution aqueuse basique, préférentiellement ladite solution ammoniacale et enfin ledit tensio-actif, préférentiellement l'ammonyl BR 1244. Ledit tensio-actif peut être soit directement introduit dans ladite solution aqueuse basique soit introduit dans ladite colonne par injection continue. Le volume de ladite colonne est constitué jusqu'à 1% volume dudit tensio-actif, jusqu'à 4 % volume d'air, de 6 à 10 % volume de ladite phase organique, le reste étant occupé par ladite phase aqueuse basique.

La vitesse de chute des gouttelettes dans la colonne est telle qu'elles conservent leur forme sphérique de manière à obtenir des billes sphériques ayant un diamètre compris entre 1 et 3 mm, de préférence entre 1,8 et 2,2 mm. Les gouttelettes, soumises au forces de Van der Walls en traversant ladite solution aqueuse, sont rigidifiées en s'agrégeant les unes aux autres. Il en résulte la formation de billes en sortie de ladite colonne. Lesdites billes sont ensuite entraînées par un flux de ladite phase aqueuse basique, préférentiellement par un flux d'ammoniaque, récupérées et séparées de ladite phase aqueuse sur un tamis. La solution aqueuse ammoniacale récupérée est avantageusement recyclée dans ladite colonne mise en oeuvre pour la mise en forme par coagulation en gouttes.
Conformément à l'étape d) du procédé de préparation du catalyseur employé dans le procédé de conversion selon l'invention, lesdites billes sont séchées dans un caisson ventilé à température ambiante puis sont séchées à l'étuve à une température comprise entre 60 et 120°C. Le séchage dans le caisson et le séchage durent chacun généralement entre 10 et 20 heures.

Les billes sont ensuite calcinées, conformément à l'étape e) du procédé de préparation du catalyseur employé dans le procédé de conversion selon l'invention, à une température comprise entre 500 et 800 °C, préférentiellement comprise entre 550 et 700 °C. La calcination dure généralement quelques heures, préférentiellement entre 3 et 5 heures.

L'unité réactionnelle pourvue dudit catalyseur sous forme de billes sphériques pour la mise en oeuvre du procédé selon l'invention fonctionne soit en lit mobile soit en, lit fixe. Lorsqu'elle fonctionne en lit fixe ou en lit mobile, le catalyseur est régénéré périodiquement et ladite unité réalise alternativement la réaction pour la production de propylène et la régénération dudit catalyseur de manière à éliminer le coke déposé à sa surface pendant la réaction. La phase de régénération comprend généralement une phase de combustion des dépôts carbonés formés sur le catalyseur, par exemple à l'aide d'un mélange air/azote ou d'air appauvri en oxygène (notamment par recirculation de fumées) ou simplement d'air, ladite phase de combustion utilise généralement une température comprise entre 400°C et 650°C, la pression étant le plus souvent voisine de la pression utilisée dans l'unité réactionnelle. Ladite phase de combustion est suivie d'une calcination sous air sec, éventuellement dilué avec de l'azote, à une température comprise entre 500 et 600°C.

L'invention sera mieux comprise à la lecture du mode de réalisation qui va suivre, celui-ci étant donné à titre illustratif, la présente invention n'est pas limitée à ce seul mode de réalisation.

Une coupe oléfinique C4 issue d'une unité de vapocraquage et telle que caractérisée précédemment est acheminée par la ligne (6) vers une unité d'hydrogénation sélective des composés poly-insaturés (22) où de l'hydrogène a également été introduit par la ligne (6a). Ladite unité d'hydrogénation sélective est opérée dans des conditions telles que l'effluent (7) résultant de la conversion des composés poly-insaturés en composés mono-oléfiniques au sein de l'unité (22) présente une teneur en composés poly-insaturés comprise entre 10 ppm et 4000 ppm, et préférentiellement entre 50 ppm et 1000 ppm.
Une coupe essence oléfinique issue d'une unité de vapocraquage et telle que caractérisée précédemment est acheminée par la ligne (8) vers une unité d'hydrogénation sélective des composés poly-insaturés (23) où de l'hydrogène a également été introduit par la ligne (8a). L'effluent (9) sortant de ladite unité (23) est introduit dans une unité de distillation (24) qui réalise la séparation d'une fraction légère (10) et d'une fraction lourde (11). Ladite fraction légère est formée essentiellement, c'est-à-dire de 10 à 40 % poids de la coupe essence initiale, de composés à 5 atomes de carbone dont au moins 60% poids de mono-oléfines en C5 (pentène et isopentène). La fraction lourde (11) est formée de composés aromatiques (benzène, toluène, xylène, ethylbenzène), oléfiniques (oléfines en C6), et cyclo-oléfiniques (dihydro-dicyclopentadiene et dérivés alkylés).
Une coupe essence issue de FCC et telle que caractérisée plus haut dans la description est acheminée par la ligne (2) vers une unité d'hydrogénation sélective des composés poly-insaturés (20) où de l'hydrogène a également été introduit par la ligne (2a). L'effluent (3) sortant de ladite unité (20) est introduit dans une unité de séparation (21) permettant d'extraire une fraction légère (4) comprenant majoritairement des composés hydrocarbonés à 5 atomes de carbone et en particulier des mono-oléfines en C5, à savoir du pentène et de l'isopentène. Une fraction lourde (5) formée de composés paraffiniques, oléfiniques et aromatiques (benzène, toluène, xylène, ethylbenzène) est également extraite en queue de l'unité (21).
Une coupe C4 issue du FCC et telle que caractérisée plus haut dans la description est acheminée par la ligne (12) vers une unité d'hydrogénation sélective des composés poly-insaturés (25) où de l'hydrogène a également été introduit par la ligne (12a). La coupe (12) peut être avantageusement éliminée des espèces soufrées réactives qui la composent avant d'être introduite dans ladite unité d'hydrogénation sélective (25). L'effluent (13) issu de l'unité (25) contient des oléfines en C4 (butène-2, isobutène, butène-1) et des paraffines en C4 (isobutane, butane).
Les effluents (7), (10), (13) et (4) sont mélangés en amont de l'unité réactionnelle (30) pour former la charge (14a) à convertir en propylène. Ladite charge est introduite dans l'unité réactionnelle (30) pourvue d'au moins un catalyseur, ladite unité fonctionnant en lit mobile. L'effluent (15) sortant de ladite unité (30) est introduite dans une unité de séparation (31) d'où quatre flux distincts sont récoltés. Le flux de tête (16) est riche en hydrogène et contient également de l'éthylène. Le flux (17) renferme le propylène recherché. Un flux (18) formé des oléfines non converties dans ladite unité (30) et des produits secondaires formés dans ladite unité (30) est en partie recyclé en amont de ladite unité (30) par la ligne (18a) tandis que le flux (18b) contenant majoritairement des paraffines en C4 et/ou en C5 constitue la purge. La charge complète, intégrant le flux (18a) recyclé, en amont de ladite unité (30) est constituée par le flux (14b).

Les exemples qui suivent illustrent l'invention.

### Exemple 1 (invention) : préparation du catalyseur C1 avant un taux de porogène égal à 2,0% massique

On prépare une émulsion en introduisant dans un bécher d'un litre 244 g d'eau, 49 g de porogène constitué par de l'isane et 2,9 g de tensio-actif constitué par le galoryl. Le mélange est placé sous agitation à 500 tr/min pendant 15 min.
On prépare une suspension en introduisant dans un bécher de 4 litres 2198 g d'eau permutée et 69 g d'acide nitrique à 59,68 % poids, le mélange étant agité à 400 trs/min pendant 5 min. 450 g de PURAL SB3 (perte au feu = à 26,10 %) sont ensuite ajoutés et le mélange {eau permutée, acide nitrique et PURAL SB3} est agité à 1600 trs/min pendant 14 min. 332 g de zéolithe ZSM-5 sous forme H de rapport Si/Al égal à 140, commercialisée par la société Zeolyst, sont ensuite ajoutés au mélange {eau permutée, acide nitrique et PURAL SB3}, le mélange résultant est agité à 1600 trs/min pendant 3 min puis l'émulsion formée d'eau, d'isane et de galoryl est ajoutée audit mélange. L'ensemble est agité sous 1600 trs/min pendant 13 min puis la vitesse d'agitation est réduite à 625 trs/min pendant 70 min. La viscosité dudit mélange est ensuite mesurée au moyen d'un rhéomètre plan-plan pour un gradient de vitesse de cisaillement de 100 s⁻¹ et est égale 270 mPa.s.

Pour la mise en forme par coagulation en gouttes, on utilise une colonne en verre de 9,4 litres. On charge ladite colonne avec 7 litres d'une solution d'ammoniaque ayant une concentration égale à 28 g/l, 0,4 litre d'une solution d'ammonyl à 1% massique et 0,7 litre d'isane. La colonne est surmontée d'un pot d'égouttage constitué de buses, chacune étant munie d'un orifice circulaire ayant un diamètre égal à 1 mm. On introduit la suspension dans ledit pot d'égouttage, le débit d'égouttage étant tel que 80 gouttelettes sont égouttées par minute et par buse. Les gouttelettes tombent ensuite dans la phase d'isane puis dans la phase d'ammoniaque à 28 g/l, l'interface phase d'isane - phase d'ammoniaque étant constituée d'ammonyl. Les billes ainsi obtenues sont placées dans un caisson ventilé à température ambiante pendant une nuit pour effectuer un premier séchage doux puis sont placées dans une étuve pendant une nuit à 100°C. Les billes séchées sont calcinées pendant 2 heures dans un four à moufle à 600°C. On obtient ainsi le catalyseur C1 dont les caractéristiques texturales et mécaniques sont données dans le tableau 1. Il présente une résistante mécanique telle que l'écrasement grain à grain (EGG) est égal à 26 N.

### Exemple 2 (invention) : préparation du catalyseur C2 avant un taux de porogène égal à 4,0% massique.

On prépare une émulsion en introduisant dans un bécher d'un litre 247 g d'eau, 99 g de porogène constitué par de l'isane et 5,9 g de tensio-actif constitué par le galoryl. Le mélange est placé sous agitation à 500 tr/min pendant 15 min.
On prépare une suspension en introduisant dans un bécher de 4 litres 2219 g d'eau permutée et 73 g d'acide nitrique à 59,68 % poids, le mélange étant agité à 400 trs/min pendant 5 min. 450 g de PURAL SB3 (perte au feu = à 26,10 %) sont ensuite ajoutés et le mélange {eau permutée, acide nitrique et PURAL SB3} est agité à 1600 trs/min pendant 14 min. 343 g de zéolithe ZSM-5 sous forme H ayant un rapport Si/Al égal à 140 et commercialisée par la société Zeolyst sont ensuite ajoutés au mélange {eau permutée, acide nitrique et PURAL SB3}, le mélange résultant est agité à 1600 trs/min pendant 3 min puis l'émulsion formée d'eau, d'isane et de galoryl est ajoutée audit mélange. L'ensemble est agité sous 1600 trs/min pendant 13 min puis la vitesse d'agitation est réduite à 625 trs/min pendant 70 min. La viscosité dudit mélange est ensuite mesurée au moyen d'un rhéomètre plan-plan pour un gradient de vitesse de cisaillement de 100 s⁻¹ et est égale à 320 mPa.s.

Pour la mise en forme par coagulation en gouttes, on utilise une colonne en verre de 9,4 litres. On charge ladite colonne avec 7 litres d'une solution d'ammoniaque ayant une concentration égale à 28 g/l, 0,4 litre d'une solution d'ammonyl à 1% massique et 0,7 litre d'isane. La colonne est surmontée d'un pot d'égouttage constitué de buses, chacune étant munie d'un orifice circulaire ayant un diamètre égal à 1 mm. On introduit la suspension dans ledit pot d'égouttage, le débit d'égouttage étant tel que 80 gouttelettes sont égouttées par minute et par buse. Les gouttelettes tombent ensuite dans la phase d'isane puis dans la phase d'ammoniaque à 28 g/l, l'interface phase d'isane - phase d'ammoniaque étant constituée d'ammonyl. Les billes ainsi obtenues sont placées dans un caisson ventilé à température ambiante pendant une nuit pour effectuer un premier séchage doux puis sont placées dans une étuve pendant une nuit à 100°C. Les billes séchées sont calcinées pendant 2 heures dans un four à moufle à 600°C. On obtient ainsi le catalyseur C2 dont les caractéristiques texturales et mécaniques sont données dans le tableau 1. Il présente une résistante mécanique telle que l'écrasement grain à grain (EGG) est égal à 16 N.

### Exemple 3 : (invention) : préparation du catalyseur C3 avant un taux de porogène égal à 7,5 % massique.

On prépare une émulsion en introduisant dans un bécher d'un litre 249 g d'eau, 187 g de porogène constitué par de l'isane et 11,2 g de tensio-actif constitué par le galoryl. Le mélange est placé sous agitation à 500 tr/min pendant 15 min.
On prépare une suspension en introduisant dans un bécher de 4 litres 2243 g d'eau permutée et 68 g d'acide nitrique à 59,68 % poids, le mélange étant agité à 400 trs/min pendant 5 min. 450 g de PURAL SB3 (perte au feu = à 26,10 %) sont ensuite ajoutés et le mélange {eau permutée, acide nitrique et PURAL SB3} est agité à 1600 trs/min pendant 14 min. 339 g de zéolithe ZSM-5 sous forme H de rapport Si/Al égal à 140 et commercialisée par la société Zeolyst sont ensuite ajoutés au mélange {eau permutée, acide nitrique et PURAL SB3}, le mélange résultant est agité à 1600 trs/min pendant 3 min puis l'émulsion formée d'eau, d'isane et de galoryl est ajoutée audit mélange. L'ensemble est agité sous 1600 trs/min pendant 13 min puis la vitesse d'agitation est réduite à 625 trs/min pendant 70 min. La viscosité dudit mélange est ensuite mesurée au moyen d'un rhéomètre plan-plan pour un gradient de vitesse de cisaillement de 100 s⁻¹ et est égale 270 mPa.s.

Pour la mise en forme par coagulation en gouttes, on utilise une colonne en verre de 9,4 litres. On charge ladite colonne avec 7 litres d'une solution d'ammoniaque ayant une concentration égale à 28 g/l, 0,4 litre d'une solution d'ammonyl à 1% massique et 0,7 litre d'isane. La colonne est surmontée d'un pot d'égouttage constitué de buses, chacune étant munie d'un orifice circulaire ayant un diamètre égal à 1 mm. On introduit la suspension dans ledit pot d'égouttage, le débit d'égouttage étant tel que 80 gouttelettes sont égouttées par minute et par buse. Les gouttelettes tombent ensuite dans la phase d'isane puis dans la phase d'ammoniaque à 28 g/l, l'interface phase d'isane - phase d'ammoniaque étant constituée d'ammonyl. Les billes ainsi obtenues sont placées dans un caisson ventilé à température ambiante pendant une nuit pour effectuer un premier séchage doux puis sont placées dans une étuve pendant une nuit à 100°C. Les billes séchées sont calcinées pendant 2 heures dans un four à moufle à 600°C. On obtient ainsi le catalyseur C3 dont les caractéristiques texturales et mécaniques sont données dans le tableau 1. Il présente une résistante mécanique telle que l'écrasement grain à grain (EGG) est égal à 26 N.

### Exemple 4 (comparatif) : préparation du catalyseur C0 en l'absence de porogène.

On prépare une suspension en introduisant dans un bécher de 4 litres 2492 g d'eau permutée et 68 g d'acide nitrique à 59,76 % poids, le mélange étant agité à 400 trs/min pendant 5 min. 450 g de PURAL SB3 (perte au feu = à 26,10 %) sont ensuite ajoutés et le mélange {eau permutée, acide nitrique et PURAL SB3} est agité à 1600 trs/min pendant 14 min. 339 g de zéolithe ZSM-5 sous forme H de rapport Si/Al égal à 140 et commercialisée par la société Zeolyst sont ensuite ajoutés au mélange {eau permutée, acide nitrique et PURAL SB3}, le mélange résultant est agité à 1600 trs/min pendant 16 min puis la vitesse d'agitation est réduite à 625 trs/min pendant 70 min. La viscosité dudit mélange est ensuite mesurée au moyen d'un rhéomètre plan-plan pour un gradient de vitesse de cisaillement de 100 s⁻¹ et est égale 270 mPa.s.

Pour la mise en forme par coagulation en gouttes, on utilise une colonne en verre de 9,4 litres. On charge ladite colonne avec 7 litres d'une solution d'ammoniaque ayant une concentration égale à 28 g/l, 0,4 litre d'une solution d'ammonyl à 1 % massique et 0,7 litre d'isane. La colonne est surmontée d'un pot d'égouttage constitué de buses, chacune étant munie d'un orifice circulaire ayant un diamètre égal à 1 mm. On introduit la suspension dans ledit pot d'égouttage, le débit d'égouttage étant tel que 80 gouttelettes sont égouttées par minute et par buse. Les gouttelettes tombent ensuite dans la phase d'isane puis dans la phase d'ammoniaque à 28 g/l, l'interface phase d'isane - phase d'ammoniaque étant constituée d'ammonyl. Les billes ainsi obtenues sont placées dans un caisson ventilé à température ambiante pendant une nuit pour effectuer un premier séchage doux puis sont placées dans une étuve pendant une nuit à 100°C. Les billes séchées sont calcinées pendant 2 heures dans un four à moufle à 600°C. On obtient ainsi le catalyseur C0, non conforme à l'invention, dont les caractéristiques texturales et mécaniques sont données dans le tableau 1.

Les caractéristiques texturales et mécaniques des catalyseurs C0, C1, C2 et C3 sont données dans le tableau 1 ci-dessous.

**Tableau 1 : caractéristiques texturales et mécaniques des catalyseurs C0, C1, C2 et C3**

| | C0 | C1 | C2 | C3 |
|---|---|---|---|---|
| surface BET (m²/g) | 326 | 321 | 329 | 323 |
| vol. poreux Hg (ml/g) | 0,30 | 0,41 | 0,47 | 0,49 |
| vol. macroporeux Hg (ml/g) | 0,005 | 0,12 | 0,18 | 0,14 |
| vol. mésoporeux Hg ( ml/g) | 0,29 | 0,29 | 0,29 | 0,35 |
| taille billes sphériques (mm) | 1,8-2,2 | 1,8-2,2 | 1,8-2,2 | 1,8-2,2 |

### Exemple 5 : Performances catalytiques des catalyseurs C0, C1, C2 et C3 dans un procédé de production de propylène à partir d'une charge constituée de butène-1 et pentène-1.

Chacun des catalyseurs C0, C1, C2 et C3 sont testés séparément dans un procédé de production de propylène équipé d'une unité pourvue d'un réacteur muni de 1,5 g de l'un desdits catalyseurs placé dans un lit de SiC de même granulométrie. Le réacteur est chauffé à 510°C. La charge alimentant ladite unité est composée de 50% en poids de butène-1 pur à 99% molaire et de 50% en poids de pentene-1. Le débit de ladite charge est égal à 6,75 g/h pour les catalyseurs C1, C2 et C3. De l'azote est également introduit dans ladite unité sous un débit égal à 6,7 l/h. Ladite unité fonctionne en lit fixe. La réaction au sein de ladite unité est réalisée sous une pression égale à 0,05 MPa. La pph est de 4,5 h⁻¹ lorsque les catalyseurs C1, C2 et C3 sont testés et est égale à 3 h⁻¹ lorsque le catalyseur C0 est testé et pour lequel le débit de la charge est de 4,5 g/h. En sortie de l'unité réactionnelle, on récolte le gaz qui est analysé au travers d'un chromatographe en phase gazeuse.

Les performances catalytiques obtenues par chacun des catalyseurs C0, C1, C2 et C3 au cours des tests décrits ci-dessus sont données dans le tableau 2.

**Tableau 2 : performances catalytiques obtenues par chacun des catalyseurs C0, C1, C2 et C3.**

| | C0 | C1 | C2 | C3 |
|---|---|---|---|---|
| pph (h⁻¹) | 3 | 4,5 | 4,5 | 4,5 |
| | | | | |
| temps sous flux (h) | 10 | 10 | 10 | 10 |
| conversion oléfines (%) | 70,5 | 70,2 | 70,7 | 70,1 |
| sélectivité propylène (%) | 31,5 | 39,6 | 39,3 | 41,6 |
| rendement propylène (%) | 22,2 | 27,8 | 27,8 | 29,1 |
| propylène/isobutène | 2,1 | 2,4 | 2,3 | 2,5 |
| | | | | |
| temps sous flux (h) | 40 | 40 | 40 | 40 |
| conversion oléfines (%) | 64,1 | 64,2 | 64,1 | 66,8 |
| sélectivité propylène (%) | 36,4 | 43,1 | 42,4 | 42,5 |
| rendement propylène (%) | 23,3 | 27,7 | 27,2 | 28,4 |
| propylène/isobutène | 1,7 | 2,0 | 2,0 | 2,2 |

La conversion en oléfines correspond à la quantité pondérale d'oléfines C4/C5 en sortie soustraite de la quantité pondérale d'oléfines C4/C5 en entrée de la zone réactionnelle, l'ensemble étant divisé par la quantité pondérale d'oléfines C4/C5 en entrée. Le rendement en propylène est calculé comme étant égal à la quantité pondérale de propylène produite rapportée à la quantité de la charge fraîche.

Les résultats figurant dans le tableau 2 montrent qu'à iso-conversion en oléfines (ppH = 4,5 h⁻¹ pour C1, C2 et C3 et pph = 3 h⁻¹ pour C0), les catalyseurs C1, C2 et C3 sont plus sélectifs envers le produit désiré, le propylène, et conduisent à un bien meilleur rendement en propylène que le catalyseur C0 qui a été préparé en l'absence de porogène.

Les tests ont été réalisés pour une durée de 10 heures et de 40 heures après le début de l'injection de la charge (temps sous flux). Malgré la désactivation observée sur chacun des catalyseurs lorsque le temps sous flux augmente (chute de conversion pour l'ensemble des catalyseurs entre les tests après 10 h d'injection de charge et les tests après 40 h d'injection de charge), la sélectivité en propylène et en conséquence le rapport propylène/isobutène sont toujours meilleurs lorsque les catalyseurs C1, C2 et C3 sont employés. que lorsque le catalyseur C0 est employé

### Exemple 6 : Performances catalytiques des catalyseurs C0, C1, C2 et C3 dans un procédé de production de propylène à partir d'une charge associant une charge issue d'une unité de vapocraquage et une charge issue du FCC.

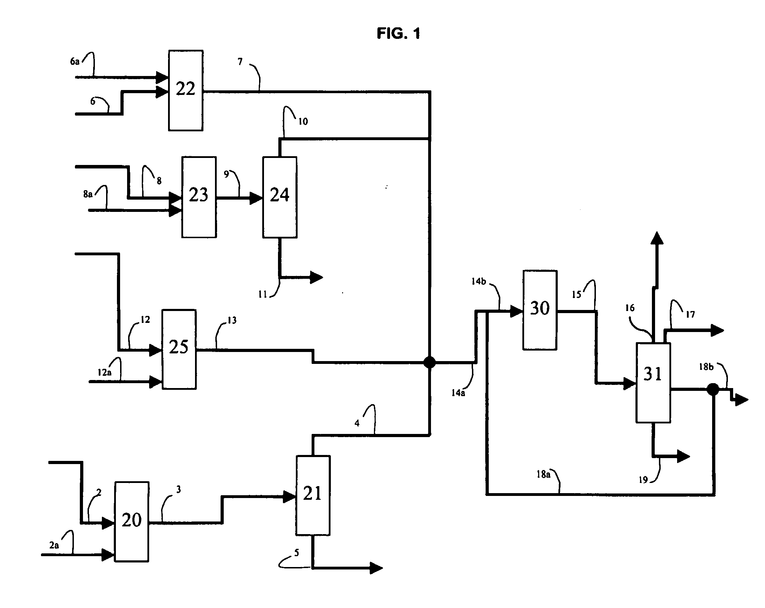

Chacun des catalyseurs C0, C1, C2 et C3 sont testés séparément dans un procédé de production de propylène équipé d'une unité pourvue d'un réacteur muni de l'un desdits catalyseurs. Chaque catalyseur est mis en oeuvre dans ledit procédé alimenté par une charge, préalablement chauffée à 510°C avant d'être introduite dans le réacteur, dont la composition est donné dans le tableau 3 (voir la composition des flux 6, 12, 2 et 8 qui correspondent à la dénomination des flux donné sur la figure 1). Le débit de chacun des flux est donné en kg/h dans le tableau 3. Ladite unité fonctionne en lit mobile. La réaction au sein de ladite unité est réalisée sous une pression égale à 0,15 MPa. La pph est de 4,5 h⁻¹. Les flux 14a et 18a figurant dans le tableau 3 correspondent à la dénomination des flux donné sur la figure 1. En sortie de l'unité réactionnelle, on récolte le gaz qui est analysé au travers d'un chromatographe en phase gazeuse.

**Tableau 3 : composition des différents flux hydrocarbonés traversant le procédé de production de propylène en présence des catalyseurs C0, C1, C2 et C3.**

| | | | | | | | | | |
|---|---|---|---|---|---|---|---|---|---|
| catalyseur | | | | | | C0 | C1 | C2 | C3 |
| n-C4= | 2340 | 3556 | | | 9672 | 11788 | 5402 | 5346 | 4413 |
| iC4= | 2889 | 1524 | | | 4413 | 6061 | 3473 | 3409 | 2897 |
| C4== | 3976 | 20 | | | 22 | 87 | 43 | 40 | 35 |
| C4 P | 795 | 4900 | | | 5893 | 30321 | 15161 | 13834 | 12314 |
| n+i C5= | | | 6000 | 6500 | 12500 | 3654 | 2549 | 2497 | 2216 |
| cycloC5 | | | 500 | 2500 | 3000 | 90 | 74 | 74 | 75 |
| C5== | | | | | | | | | |
| C5 P | | | 3500 | 1000 | 4500 | 20046 | 10087 | 9211 | 8140 |
| C6-C12 | | | 50000 | 30000 | | 10487 | 8740 | 8652 | 8099 |
| total (kg/h | 10000 | 10000 | 60000 | 40000 | 40000 | 82533 | 45530 | 43062 | 38188 |

| | | | | | | | | | |
|---|---|---|---|---|---|---|---|---|---|
| où nC4= est le n-butène, iC4= est l'isobutène, C4== est le butadiène, C4 P sont les paraffines en C4, n+iC5= sont les n- et iso-pentènes, cycloC5 est le cycolpentène, C5== est le pentadiène, C5 P sont les paraffines en C5 et le cyclopentane, C6-C12 sont les hydrocarbures (paraffines, oléfines, aromatiques) ayant de 6 à 12 atomes de carbone par molécule. | | | | | | | | | |

Les tests sont réalisés pour un temps de cycle de 48 heures, le temps de cycle correspondant à la durée pendant laquelle le catalyseur est en contact avec la charge pour réaliser la réaction de production de propylène. Après cette durée de 48 heures, chaque catalyseur est régénéré.

Les performances catalytiques obtenues par chacun des catalyseurs C0, C1, C2 et C3 au cours des tests décrits ci-dessus sont données dans le tableau 4.

**Tableau 4 : performances catalytiques obtenues par chacun des catalyseurs C0, C1, C2 et C3.**

| catalyseur | C0 | C1 | C2 | C3 |
|---|---|---|---|---|
| conversion oléfines (%) | 79,2 | 77,5 | 75,3 | 76,3 |
| sélectivité propylène (%) | 39,0 | 39,9 | 41,0 | 40,5 |
| rendement propylène (%) | 30,9 | 30,9 | 30,9 | 30,9 |
| taux de recycle | 2,1 | 1,1 | 1,1 | 1,0 |

La conversion en oléfines correspond à la quantité pondérale d'oléfines C4/C5 en sortie soustraite de la quantité pondérale d'oléfines C4/C5 en entrée de la zone réactionnelle avant le recyclage du flux (18a), l'ensemble étant divisé par la quantité pondérale d'oléfines C4/C5 en entrée. Le rendement en propylène est calculé comme étant égal à la quantité pondérale de propylène produite rapportée à la quantité de la charge fraîche.

Les résultats figurant dans le tableau 4 montrent que les procédés mis en oeuvre en présence des catalyseurs C1, C2 et C3 conduisent à un rendement en propylène identique à celui obtenu par le procédé mis en oeuvre par le catalyseur C0 alors que le débit du flux (18a) recyclé est deux fois plus faible que celui employé pour la mise en oeuvre du procédé utilisant le catalyseur C0. Diminuer de moitié le taux de recycle lorsque le catalyseur utilisé dans le procédé de production de propylène est préparé en présence de porogène permet de diminuer sensiblement la consommation énergétique globale des procédés mis en oeuvre en présence des catalyseurs C1, C2 et C3. De plus, la sélectivité envers le propylène est accrue lorsque les catalyseurs sont préparés en présence de porogène.

## Revendications

1. Procédé de conversion directe d'une charge hydrocarbonée comprenant au moins des oléfines ayant 4 atomes de carbone et au moins des oléfines ayant 5 atomes de carbone pour la production de propylène, ledit procédé comprenant le passage de ladite charge dans au moins une unité réactionnelle pourvue d'au moins un catalyseur se présentant sous forme de billes sphériques de diamètre compris entre 1 et 3 mm, chacune desdites billes sphériques comprenant au moins une zéolithe et au moins un support à base d'alumine et présentant une distribution poreuse telle que le volume macroporeux mesuré par porosimétrie au mercure est compris entre 0,10 et 0,20 ml/g et le volume mésoporeux mesuré par porosimétrie au mercure est compris entre 0,25 et 0,35 ml/g.

2. Procédé de conversion directe selon la revendication 1 tel que chacune desdites billes sphériques présente des domaines macroporeux au delà de 50 nm.

3. Procédé de conversion directe selon la revendication 1 ou la revendication 2 tel que ledit catalyseur se présente sous forme de billes sphériques de diamètre compris entre 1,8 et 2,2 mm.

4. Procédé de conversion directe selon l'une des revendications 1 à 3 tel que ladite zéolithe présente dans chacune desdites billes sphériques est choisie parmi les zéolithes de type structural MEL, MFI, NES, EUO, FER, CHA, MFS, MWW et NES.

5. Procédé de conversion directe selon la revendication 4 tel que ladite zéolithe est une zéolithe de type structural MFI.

6. Procédé de conversion directe selon l'une des revendications 1 à 5 tel que ledit catalyseur est préparé selon un procédé comprenant a) la préparation d'au moins une émulsion formée d'au moins un porogène, de l'eau et d'un tensioactif, ledit porogène étant une coupe kérosène paraffinique ayant de 10 à 14 atomes de carbone, formée de normale et d'iso-paraffines, et présentant un point d'ébullition compris entre 220 et 350°C b) la préparation d'une suspension formée d'eau, d'acide, d'une source d'alumine, d'au moins une zéolithe et de ladite émulsion préparée au cours de l'étape a), c) la mise en forme par coagulation en gouttes consistant i) à faire passer ladite suspension formée en b) dans un pot d'égouttage constitué de buses ayant chacune un orifice de taille calibrée de manière à former des gouttelettes, ii) à faire passer, selon un mouvement descendant, lesdites gouttelettes dans une colonne contenant une phase supérieure constituée par une phase organique et une phase inférieure constituée par une phase aqueuse basique, l'interface phase organique - phase aqueuse étant constitué d'un tensio-actif, de manière à récolter des billes sphériques, d) le séchage desdites billes et e) la calcination desdites billes.

7. Procédé de conversion directe selon la revendication 6 tel que le taux de porogène égal à la masse de porogène sur la masse de l'eau engagée dans l'émulsion et de l'eau engagée dans la suspension est compris entre 1,5 et 8 % massique.

8. Procédé de conversion directe selon la revendication 6 ou la revendication 7 tel que le taux d'acide, engagé dans la suspension, égal au produit de la concentration (% poids) dudit acide par la masse dudit acide rapporté à la masse sèche de la source d'alumine est compris entre 10 et 15 % massique.

9. Procédé de conversion directe selon l'une des revendications 1 à 8 tel que ladite charge hydrocarbonée provient soit de la coupe oléfinique C4/C5 issue d'une unité de vapocraquage soit des coupes oléfiniques C4 et essence issues d'une unité de craquage catalytique fluide (FCC) soit encore d'un mélange desdites coupes issues du vapocraquage et du craquage catalytique fluide..

10. Procédé de conversion directe selon l'une des revendications 1 à 9 tel que ladite unité réactionnelle réalisant la conversion de ladite charge hydrocarbonée pour la production de propylène et pourvue d'au moins dudit catalyseur est mise en oeuvre à une température comprise entre 450 et 580°C, à une pression opératoire comprise entre 0,01 et 0,5 MPa et une pph (débit massique horaire de charge / masse du catalyseur) comprise entre 1 et 20 **h⁻¹.**

11. Procédé de conversion directe selon l'une des revendications 1 à 10 tel que ledit catalyseur est mis en oeuvre dans ladite unité réactionnelle fonctionnant soit en lit mobile soit en lit fixe.

## Claims

1. A process for once-through conversion of a hydrocarbon feed comprising at least olefins containing 4 carbon atoms and at least olefins containing 5 carbon atoms for the production of propylene, said process comprising passing said feed into at least one reaction unit provided with at least one catalyst in the form of spherical beads with a diameter in the range 1 to 3 mm, each of said spherical beads comprising at least one zeolite and at least one alumina-based support and having a pore distribution such that the macroporous volume, measured by mercury porosimetry, is in the range 0.10 to 0.20 ml/g and the mesoporous volume, measured by mercury porosimetry, is in the range 0.25 to 0.35 ml/g.

2. A once-through conversion process according to claim 1, in which each of said spherical beads has macroporous domains of more than 50 nm.

3. A once-through conversion process according to claim 1 or claim 2, in which said catalyst is in the form of spherical beads with a diameter in the range 1.8 to 2.2 mm.

4. A once-through conversion process according to one of claims 1 to 3, in which said zeolite present in each of said spherical beads is selected from zeolites with structure type MEL, MFI, NES, EUO, FER, CHA, MFS, MWW and NES.

5. A once-through conversion process according to claim 4, in which said zeolite is a zeolite with structure type MFI.

6. A once-through conversion process according to one of claims 1 to 5, in which said catalyst is prepared using a process comprising a) preparing at least one emulsion formed from at least one porogen, water and a surfactant, said porogen being a paraffinic kerosene cut containing 10 to 14 carbon atoms, formed from normal and iso paraffins, and with a boiling point in the range 220°C to 350°C, b) preparing a suspension formed from water, acid, a source of alumina, at least one zeolite and said emulsion prepared during step a), c) forming by drop coagulation, consisting of i) passing said suspension formed in b) into a draining pot constituted by nozzles each having an orifice calibrated to form droplets, ii) passing, in a downward movement, said droplets into a column containing an upper phase constituted by an organic phase and a lower phase constituted by a basic aqueous phase, the organic phase - aqueous phase interface being constituted by a surfactant, to harvest spherical beads, d) drying said beads and e) calcining said beads.

7. A once-through conversion process according to claim 6, in which the porogen content, equal to the mass of porogen over the mass of water engaged in the emulsion and the water engaged in the suspension, is in the range 1.5% to 8% by weight.

8. A once-through conversion process according to one of claims 6 or 7, in which the amount of acid engaged in the suspension is equal to the product of the concentration (% by weight) of said acid and the mass of said acid with respect to the dry mass of the alumina source, is in the range 10% to 15% by weight.

9. A once-through conversion process according to one of claims 1 to 9, in which said hydrocarbon feed derives either from the C4/C5 olefinic cut from the steam cracking unit or from olefinic C4 cuts and gasoline derived from a fluid catalytic cracking (FCC) unit or from a mixture of said cuts derived from steam cracking and fluid catalytic cracking.

10. A once-through conversion process according to one of claims 1 to 10, in which said reaction unit carrying out the conversion of said hydrocarbon feed for the production of propylene and provided with at least one said catalyst is carried out at a temperature in the range 450°C to 580°C, at an operating pressure in the range 0.01 to 0.5 MPa and at an HSV (ratio of the hourly volume flow rate of fresh liquid feed over the loaded volume of catalyst) in the range 1 to 20 h⁻¹.

11. A once-through conversion process according to one of claims 1 to 11, in which said catalyst is used in said reaction unit operating either in moving bed mode or in fixed bed mode.

## Patentansprüche

1. Verfahren zur direkten Umwandlung einer Kohlenwasserstoffcharge, die mindestens Olefine mit 4 Kohlenstoffatomen und mindestens Olefine mit 5 Kohlenstoffatomen umfasst, zur Herstellung von Propylen, wobei das Verfahren das Leiten der Charge in mindestens eine Reaktionseinheit umfasst, die mit mindestens einem Katalysator versehen ist, der in Form von Kugeln mit einem Durchmesser zwischen 1 und 3 mm vorliegt, wobei jede der Kugeln mindestens ein Zeolith und mindestens einen Träger auf der Basis von Aluminiumoxid umfasst und eine solche Porenverteilung aufweist, dass das durch Quecksilberporosimetrie gemessene Makroporenvolumen zwischen 0,10 und 0,20 ml/g liegt und das durch Quecksilberporosimetrie gemessene Mesoporenvolumen zwischen 0,25 und 0,35 ml/g liegt.

2. Verfahren zur direkten Umwandlung nach Anspruch 1, wobei jede der Kugeln makroporöse Bereiche jenseits von 50 nm aufweist.

3. Verfahren zur direkten Umwandlung nach Anspruch 1 oder Anspruch 2, wobei der Katalysator in Form von Kugeln mit einem Durchmesser zwischen 1,8 und 2,2 mm vorliegt.

4. Verfahren zur direkten Umwandlung nach einem der Ansprüche 1 bis 3, wobei das in jeder der Kugeln vorhandene Zeolith aus den Zeolithen vom Strukturtyp MEL, MFI, NES, EUO, FER, CHA, MFS, MWW und NES ausgewählt ist.

5. Verfahren zur direkten Umwandlung nach Anspruch 4, wobei das Zeolith ein Zeolith vom Strukturtyp MFI ist.

6. Verfahren zur direkten Umwandlung nach einem der Ansprüche 1 bis 5, wobei der Katalysator nach einem Verfahren hergestellt wird, welches umfasst: a) die Herstellung mindestens einer Emulsion, die von mindestens einem Porogen, Wasser und einem Tensid gebildet wird, wobei das Porogen eine paraffinische Kerosinfraktion mit 10 bis 14 Kohlenstoffatomen ist, die von normalem Paraffin und Isoparaffinen gebildet wird und einen Siedepunkt zwischen 220 und 350 °C aufweist; b) die Herstellung einer Suspension, die von Wasser, Säure, einer Aluminiumoxidquelle, mindestens einem Zeolith und der im Verlaufe des Schrittes a) hergestellten Emulsion gebildet wird; c) die Formgebung durch Koagulation in Tropfen, darin bestehend, i) die in b) gebildete Suspension in ein Abtropfgefäß zu leiten, das aus Düsen besteht, die jeweils eine Öffnung von kalibrierter Größe aufweisen, so dass Tröpfchen gebildet werden, ii) die Tröpfchen in einer Abwärtsbewegung in eine Kolonne zu leiten, die eine aus einer organischen Phase bestehende obere Phase und eine aus einer basischen wässrigen Phase bestehende untere Phase enthält, wobei die Grenzfläche zwischen organischer Phase und wässriger Phase aus einem Tensid besteht, so dass Kugeln gewonnen werden; d) die Trocknung der Kugeln; und e) die Kalzinierung der Kugeln.

7. Verfahren zur direkten Umwandlung nach Anspruch 6, wobei der Porogenanteil, der gleich dem Verhältnis der Masse des Porogens zur Masse des in der Emulsion enthaltenen Wassers und des in der Suspension enthaltenen Wassers ist, zwischen 1,5 und 8 Massenprozent liegt.

8. Verfahren zur direkten Umwandlung nach Anspruch 6 oder Anspruch 7, wobei der Anteil der in der Suspension enthaltenen Säure, der gleich dem Produkt der Konzentration (Gew.-%) der Säure mit der Masse der Säure ist, bezogen auf die Trockenmasse der Aluminiumoxidquelle, zwischen 10 und 15 Massenprozent liegt.

9. Verfahren zur direkten Umwandlung nach einem der Ansprüche 1 bis 8, wobei die Kohlenwasserstoffcharge entweder aus der Olefinfraktion C4/C5 hervorgegangen ist, die aus einer Dampfcrackanlage stammt, oder aus den Olefinfraktionen C4 und der Benzinfraktion, die von einer Anlage zum katalytischen Cracken im Fließbettverfahren (Fluid Catalytic Cracking, FCC) stammen, oder auch aus einer Mischung dieser vom Dampfcracken und vom katalytischen Cracken im Fließbettverfahren stammenden Fraktionen.

10. Verfahren zur direkten Umwandlung nach einem der Ansprüche 1 bis 9, wobei die Reaktionseinheit, welche die Umwandlung der Kohlenwasserstoffcharge für die Herstellung von Propylen realisiert und mit mindestens dem Katalysator versehen ist, mit einer Temperatur zwischen 450 und 580 °C, mit einem Betriebsdruck zwischen 0,01 und 0,5 MPa und einem pph (Massendurchsatz der Charge pro Stunde /Masse des Katalysators) zwischen 1 und 20 h⁻¹ betrieben wird.

11. Verfahren zur direkten Umwandlung nach einem der Ansprüche 1 bis 10, wobei der Katalysator in der Reaktionseinheit entweder im Fließbett oder im Festbett eingesetzt wird.
